# EUROPEAN PATENT SPECIFICATION

(11) **EP 3 687 602 B1**
(45) Date of publication and mention of the grant of the patent: **25.02.2026**
(21) Application number: 18862870.5
(22) Date of filing: 25.09.2018
(51) Int. Cl.: A61M 5/168, A61M 5/142, A61M 5/158

(54) **FLUID DELIVERY DEVICE WITH LEAK DETECTION**
FLÜSSIGKEITSABGABEVORRICHTUNG MIT LECKDETEKTION
DISPOSITIF D'ADMINISTRATION DE LIQUIDE À DÉTECTION DE FUITE

(30) Priority: 27.09.2017 US 201762563860 P
(43) Date of publication of application: 05.08.2020
(73) Proprietor: Becton, Dickinson and Company, Franklin Lakes, NJ 07417-1880 (US)
(72) Inventor: POLITIS, Victor Isaac, Framingham, Massachusetts 01701 (US); SIEWIOREK, Gail, Wincester, Massachusetts 04890 (US)
(74) Representative: dompatent
(86) International application number: PCT/US2018/052535
(87) International publication number: WO 2019/067386

(56) References cited:
- EP-A1- 2 272 559
- US-A1- 2003 009 131
- US-A1- 2011 144 574
- US-A1- 2012 053 514
- US-A1- 2013 066 285
- US-A1- 2014 100 522
- US-A1- 2014 100 522
- US-A1- 2017 188 916

## Description

### Field of the Invention

The present invention is directed to a fluid delivery device having a leak detection member that is able to detect leakage at an injection site or delivery site on the patient. In one embodiment, the fluid delivery device is an insulin infusion set or patch pump that exhibits a visual color change in response to leakage at an injection site or a delivery site.

### Background

There are two primary methods of daily insulin therapy for patients with diabetes. The first includes syringes and insulin pens. These devices are simple to use and are relatively low in cost, but they require a needle stick at each injection, typically three to four times per day. The second mode includes insulin infusion therapy, which utilizes an insulin pump. Infusion pumps, although more complex and expensive than syringes and pens, offer the advantages of continuous infusion of insulin through a cannula such as an infusion cannula, precision dosing, and programmable delivery schedules.

The use of an infusion pump requires the use of a disposable component, typically referred to as an infusion set, line set, extension set or pump set, which conveys the insulin from a reservoir within the pump into the skin of the user. An infusion set typically consists of a pump connector, a length of tubing, and a hub or base from which an infusion cannula extends, The cannula is typically an infusion needle or a flexible catheter that is inserted into the patient. The hub or base has an adhesive which retains the base on the skin surface during use, and which may be applied to the skin manually or with the aid of a manual or automatic insertion device. In most cases, a detachable fluid connector is provided to allow the pump tubing to be disconnected from the hub or base of the infusion set when the user wishes to shower, bathe, or swim.

A second method of providing insulin infusion therapy is by a patch pump. A patch pump is a self-contained device incorporating an insulin reservoir, pump, and cannula in a single housing that can be adhered to the user's skin. A patch pump offers the advantage of not requiring the user to disconnect pump tubing when the user wishes to shower, bathe, or swim.

A problem with infusion sets and patch pumps occurs when the cannula separates from the skin of the patient or becomes dislodged such that leakage occurs at the infusion site. Leakage at the delivery site can go unnoticed by the patient and can result in high blood sugar levels. Infusion pumps generally dispense insulin in small volumes for long periods of time so that the leakage is often not noticed by the patient for an extended length of time, which can result in an improper dosage.

Accordingly, there is a continuing need in the industry for improved delivery device such as infusion sets and patch pumps that provide adequate leak detection to the patient.

EP 2 272559 A1 discloses a medical infusion device which is inserted into the subcutaneous or intramuscular area of a patient.

US 2014/0100522 A1 discloses an infusion site leak detection device.

### Summary of the Invention

The present invention is directed to a fluid infusion set having a leak detection system particularly suitable to detect the leakage of the injected fluid or substance at a delivery, infusion or injection site and provide a visual indication to the user.

Accordingly, one feature of the present invention is to provide a fluid infusion set having a leak detection component for providing a signal or indication to the patient that leakage at the delivery site has occurred.

Another feature of the invention is to provide an infusion set that provides the patient with a visible indicator that leakage has occurred or is currently occurring at an infusion site. The visible indicator is based on a color change of a color indicator component or material at or near the injection site when the indicator contacts fluid leaking from the injection site. An infusion set or patch pump in an embodiment of the invention includes a cannula for penetrating the skin of a patient for delivering a drug or other pharmaceutical agent such as insulin at an injection site. A leak detector is provided around or near the cannula where the detector undergoes a color change as a result of a chemical reaction with one or more compounds in the drug or pharmaceutical agent with one or more compounds or components on or associated with the leak detector. The leak detector contains one or more pH sensitive chemical compounds that can undergo a rapid color change when contact is made with the liquid or fluid containing the drug or pharmaceutical agent being delivered to the patient. The leak detector is located in proximity to the cannula to contact fluid leaking from the infusion site.

The leak detector is positioned in a location on one or more surfaces of the infusion set where the drug-containing liquid or fluid leaking from the delivery site can come into contact with the leak detector. The leak detector is positioned to provide a rapid detection of the leakage from the infusion site. In one embodiment, the delivery device has a bottom face for supporting the leak detector proximate the delivery site.

The infusion set has a base with a bottom face for attaching to the skin of the patient. A center portion of the base has a recessed area and a cannula extending from the base through the recessed area so that the recessed area surrounds the infusion site when attached to the patient. A leak detector is provided in the recessed area where the leak detector includes at least one pH sensitive compound that is capable of undergoing a chemical or color change when contacted with a drug or pharmaceutical agent. The color change leak detector is visible to the patient without the need for photometric or color detecting devices. The leak detector surrounds the cannula and can be spaced from the cannula a distance to define a cavity or open area for capturing and retaining the drug or pharmaceutical agent leaking from the infusion site. The infusion set or patch pump can include a clear, transparent portion or window to visualize the color change occurring in the leak detector at the infusion site.

The leak detector is a pH sensitive compound or pH indicator that is able to exhibit a color change when contacting insulin or other fluid being delivered to a patient where the insulin solution or other fluid has a pH in a range to produce a color change in the pH indicator. In one embodiment, the pH indicator is bromothymol blue or other pH sensitive compound that is able to provide a visual color change when the insulin solution or other fluid being delivered contacts the pH indicator. The insulin is generally in the form of a solution that can include stabilizers, such as phenolic stabilizers. The insulin solution has a pH that will produce a visible color change when an amount of the insulin contacts the pH indicator.

The features of the invention are further attained in one embodiment by providing an insulin delivery device comprising an insulin supply source containing insulin. An infusion set in one embodiment is adapted for penetrating the skin of a patient and having an interface for contacting skin of the patient. The interface region has a leak detector that includes a pH color indicator, such as bromothymol blue, in an amount effective to produce a visible color change when contacted with the insulin or other fluid being delivered to the patient contacts the pH color indicator.

The features of the invention are also attained by providing an infusion set comprising an insulin supply source containing insulin and an infusion set coupled to the supply source and adapted for penetrating skin of a patient. The infusion set in one embodiment has a base with an interface for attaching to skin of a patient, a cavity formed in the interface, a cannula in the cavity, and a leakage detector within the cavity and surrounding the cannula. The leakage detector has at least one pH indicator capable of undergoing a color change upon contact with the insulin or a component contained in the insulin and is visible through the delivery device.

A not-claimed method of detecting leakage at an injection delivery site of a patient positions an injection delivery device in an injection site of the patient where the delivery device has a delivery member and a pH indicator adapted for exhibiting a color change in contact with leakage of a fluid from the delivery site on the patient. The delivery device in one embodiment includes a cannula such as a flexible catheter that is introduced into the patient for delivering the fluid to the patient. The pH indicator can be positioned next to or around the cannula or catheter to contact fluid leakage at the injection site.

The additional features of the invention are attained by providing a method of detecting a leak between an insulin infusion set and a point of delivery to a patient. The method comprises providing a leak detector at the point of delivery. The leak detector in one embodiment includes bromoti-ivmol blue in an amount sufficient to produce a color change upon contact with insulin leaking from the point of delivery.

These and other advantages and salient features of the invention will become apparent from the annexed drawings and the following detailed description of the invention which disclose various embodiments of the present invention.

### Brief Description of the Drawings

The following is a brief description of the drawings, in which:
Fig. 1 is a perspective view of an insulin infusion set in one embodiment of the invention;
Fig. 2 is an exploded view of the infusion set of Fig. 1 showing the supply coupling being disconnected;
Fig. 3 is a perspective view of the base of the infusion set;
Fig. 4 is a bottom view of the infusion set of Fig. 3;
Fig. 5 is a side view of the infusion set showing the flexible cannula as a flexible catheter;
Fig. 6 is a cross-sectional view of the infusion set of Fig. 5 showing the leak detector;
Fig. 7 is a cross-sectional view of the infusion set showing the leak detector in another embodiment, and
Fig. 8 is a cross-sectional view of the infusion set showing the leak detector in a further embodiment.

### Detailed Description of the Embodiments

The present invention is directed to a fluid infusion set having a leak detection system such as a fluid delivery device or an insulin delivery device. The invention is particularly directed to a fluid delivery device having a leak detector that provides a visual indication of leakage at the injection site. The invention is further directed to a fluid delivery device for delivering a fluid containing an active agent to a patient. The fluid delivery device is typically a drug delivery device for delivering a drug such as insulin. In the disclosure, the injection site can be used interchangeably with the infusion site or delivery site on the patient for the substance being delivered to the patient.

Reference is made to embodiments of the present invention, which are illustrated in the accompanying drawings, wherein like reference numerals refer to like elements throughout. The embodiments described herein exemplify, but do not limit, the present invention by referring to the drawings. The exemplary embodiments are presented in separate descriptions, although the individual features and construction of these embodiments can be combined in any number of ways to meet the therapeutic needs of the user,

This disclosure is not limited in its application to the details of construction and the arrangement of components set forth in the following description or illustrated in the drawings. The embodiments herein are capable of being modified, practiced or carried out in various ways. Also, it will be understood that the phraseology and terminology used herein is for the purpose of description and should not be regarded as limiting. The use of "including," "comprising," or "having" and variations thereof herein is meant to encompass the items listed thereafter. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as up, down, bottom, and top are relative, and are to aid illustration, but are not limiting. The embodiments are not intended to be mutually exclusive so that the features of one embodiment can be combined with other embodiments as long as they do not contradict each other.

The infusion set is for connecting to an infusion pump or other supply device as known in the industry. In a not-claimed example, the delivery device can be a self-contained patch pump having an internal drug reservoir. The drug delivery device in the illustrated embodiment of the invention includes a fluid supply shown as an infusion set 12 connected to an infusion pump by a conduit or supply tube 16. The infusion set is primarily for the delivery of insulin to a patient at a controlled rate and dosage. Other fluids can also be administered by infusion to the patient such as HIV drugs, drugs to treat pulmonary hypertension, pain medications, anti-cancer treatments, vitamins, growth hormones, or other substances.

The delivery device generally includes a cannula, such as a flexible catheter, needle or other device that is positioned in or below the skin of the patient to a depth to provide sufficient delivery to the patient. The delivery device includes a pH indicator that complements the pH of the fluid delivered to the patient to provide a visible color change to the pH indicator in the presence of the fluid. In one embodiment, the pH indicator can have first color or be colorless when in contact with the skin of the patient and produces a different color when in contact with the fluid.

The delivery device is an infusion set or patch pump (not claimed). The delivery device has an area that can be an open area or chamber for receiving and/or capturing fluids leaking at the injection site and producing a visible color change. The fluid delivery device for introducing a fluid to a patient includes a fluid supply for delivering a fluid containing an active agent and a stabilizing or preserving agent, a delivery element for penetrating the skin and a leak detector. The injection site or infusion site refers to the location on the patient where the insulin or other fluid is delivered to the patient. In the embodiments shown, the injection site is the point of penetration of the cannula into skin of the patient.

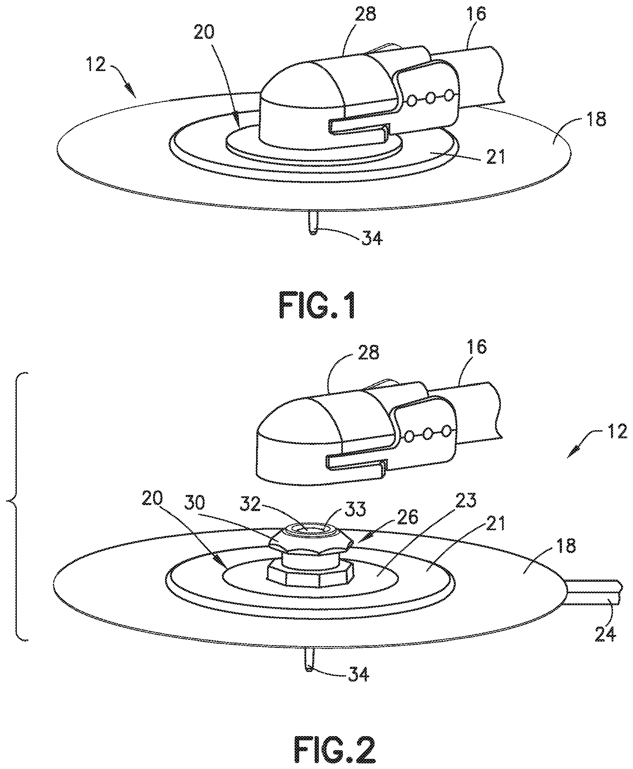

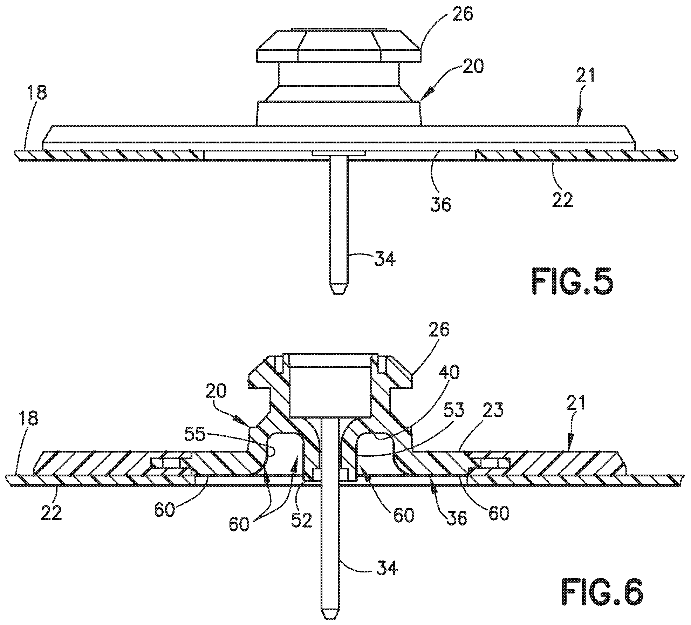

Referririg to Figs. 1-4 of the drawings, the infusion set 12 has a flexible pad 18, a centrally located hub 20 for connecting to a fluid supply, and a base 21. The pad 18 is made of a sufficiently flexible material to conform to the patient's skin when attached. The bottom face of the pad 18 includes an adhesive 22 for attaching the pad and hub to the patient shown in Figs. 5 and 6. A protective peel layer 24 shown in Fig. 2 is provided to cover the adhesive 22 during storage and which can be removed by the patient at the time of use.

The hub 20 is centrally located and attached to the pad 18 to provide a substantially fluid-tight seal between the pad and hub. The central hub 20 has a top face 23 with a fluid port 26 for connecting to a detachable fluid coupling 28. In one embodiment, hub 20 and/or the base 21 can be made partially or entirely from a clear or sufficiently transparent or translucent plastic material where the patient can visualize and observe a color change in the leak detector between the hub 20 and the skin of the patient. The transparent portion or area can be on an upper face 23 of the hub 20 or pad that is easily viewed by the patient. Port 26 has a substantially cylindrical configuration with an outwardly extending annular flange 30. The annular flange 30 connects to a corresponding recess in the coupling 28 as shown in Fig. 1. An axial passage 32 is sealed near its upper end by a pre-slit resilient septum 33 and extends through the port 26 and hub 20 for supplying the fluid to the patient. A blunt plastic cannula of the fluid connector 28 penetrates the septum 33 to establish fluid flow to the infusion set 12.

A delivery element, such as a cannula 34 or other delivery element for penetrating or positioning in the skin at the delivery site is coupled to the hub 20 and extends from an interface region on a bottom face 36 of the hub 20 as shown in Figs. 1, 2, and 5. The cannula 34 can be a rigid cannula made of stainless steel or a soft flexible cannula or flexible catheter as known in the art. The soft flexible cannula typically includes an insertion needle (not shown) that is able to penetrate the skin to position the flexible cannula into the skin after which the insertion needle is removed. Typically, the infusion set will have a flexible cannula and an insertion needle as known in the art.

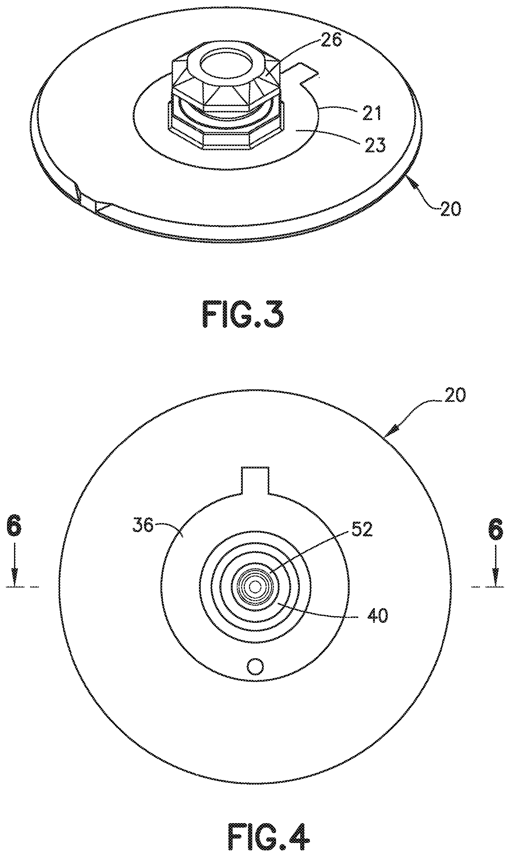

The bottom face 36 of the hub 20 as shown in the embodiment of Figs. 4 and 6 includes a recess 40, a cannula support post 52 and the cannula 34. The recess 40 in the embodiment shown has a substantially annular shape for forming an open area or cavity in the bottom face of the hub 20. The recess 40 in the embodiment shown is formed around the cannula support post 52 and around the cannula 34. Positioned on or within the hub 20 is a leak indicator that is able to provide a visible color change to the patient when the fluid being delivered to the patient contacts the leak detector. In one embodiment, the color change is visible to the patient through the top face 23 of the hub 20.

The leak indicator includes at least one pH sensitive compound or pH indicator compound that is stable during normal operation and use of the delivery device and is able to exhibit a visual color change when the fluid leaking from the injection site contacts the pH indicator. The leak detector can include a mixture of pH indicators that can improve the detection of the insulin. The pH indicator is sensitive to the pH of the insulin or other fluid to undergo a color change relative to the color before contact with the fluid leakage. The pH indicator is selected based on the pH range of the insulin solution or other fluid to provide a visual color change when the fluid contacts the pH indicator. The amount of the pH indicator is present in an amount to detect fluid leakage in amounts that are considered significant that will reduce the effectiveness or treatment of the patient. In one embodiment, the pH indicator is present in an amount that will detect insulin above a threshold amount corresponding to a leakage amount that prevents proper insulin delivery. For example, the leak indicator and the pH indicator are present that will minimize detecting very small amounts of fluid leakage that do not interfere with the treatment of the patient and thereby reduce the risk of false indication that significant leakage has occurred or presently occurring. In one embodiment, the pH indicator is able to detect leakage of at least about 100 µl or more. The amount of the pH indicator compound can be present in an amount of about 0.5g to about 25g depending on the location of the leak detector relative to the delivery site.

A particularly suitable pH indicator is bromothymol blue that is able to undergo a color change in contact with the insulin solution. Bromothymol blue is normally yellow at acidic condition, green at neutral conditions, and blue at alkaline conditions. The pH of skin is normally pH 4.7 to 6.4 particularly after washing with soap. Commercially available insulin is generally pH 7.0 to 7.8 that produces a color change to bromothymol blue. Examples of commercially available insulin are available under the tradenames Lispro, Aspart and Gluisine.

Bromothymol blue when used as the pH indicator is generally yellow or green during normal use when the infusion set and the pH indicator are in contact the skin of the patient. Bromothymol blue generally does not exhibit a color change when contacting the skin or water. The pH indicator is selected to avoid a reaction with the skin of the patient to inhibit or reduce the occurrence of false positive indication of leakage. Bromothymol blue exhibits a green color when the pH indicator contacts the insulin solution at pH of 7.0 to 7.8. Other pH color indicators can also be used that are able to provide a suitable color change before and after contact with insulin. The pH color indicators in the embodiments of the invention are able to detect the pH of the insulin solution provided by one or more components or diluents of the insulin solution. Other examples of pH indicators that be exhibit a visible color change in the presence of insulin solutions having a pH of about 7.0 to 7.8 in addition to or as an alternative to bromothyrnol blue include phenol red, neutral red, cresol red, and naphthophthalein. The pH indicator in one embodiment produces a color change at pH 7.0 to pH 7.8 to indicate the leakage of an insulin solution.

In the embodiment of Figs. 3-6, the pH indicator is applied to one or more surfaces of the bottom side of the hub 20 where the insulin or other fluid leaking from the delivery site is able to contact the pH indicator. In the embodiments shown, the pH indicator is applied as a coating directly onto one or more surfaces of the hub 20 and/or the base 21. The coating can be applied as a solution by suitable coating procedures for use with plastic materials. Preferably the coating is dry, stable until ready for use, and adheres sufficiently to the selected surface of the hub to prevent transfer or wear to another surface or to the skin of the patient during normal use of the delivery device.

In the embodiment of Figs. 3-6 the leak detector is a pH indicator 60 and is applied as a coating to one or more locations of the hub in an area next to and/or surrounding the post 52 and the cannula 34. The coating contains an amount of the pH indicator to be able to detect amounts of leakage that interfere with the desired dosage over a desired period of time. In one embodiment, the coating contains about 0.5g or more of the pH indicator on the delivery device. The pH indicator 60 in one embodiment can be applied to the side wall 53 of the post 52 within the recess, the outer annular face 55 of the recess, and/or on the bottom face 36 of the hub 20 facing the skin of the patient as shown in Fig. 6. In one embodiment, the pH indicator 60 is provided on the face of the hub that contacts the skin surrounding the cannula and is spaced radially inwardly from the adhesive 22. The adhesive 22 preferably contacts the skin of the patient to attach the device to the patient during use and form a fluid seal around the cannula hub and cannula. The adhesive can form the liquid seal around the delivery site to contain fluid leaking from the delivery site within the open area defined by the adhesive and direct or contain the fluid in the location of the pH indicator to promote rapid visualization of the color change and leakage at the delivery site. In the embodiment shown, at least a portion of the hub 20 and/or base 21 is sufficiently transparent where the color change of the pH indicator is visible through the top face 23 of the hub 20.

The leak indicator in the embodiments of the device is a pH color indicator that is able to change color and provide a visual indication to the patient that fluid is or may be leaking from the delivery site around the cannula as a result of improper placement of the cannula or accidental movement and withdrawal of the catheter during use. The pH indicator can be applied as a coating on at least one surface of the hub in a location that is able to come in contact with fluid leaking from the injection site. The coating of the pH indicator can be applied by any suitable coating method that can apply the coating to the plastic material of the hub. The coating is typically applied in a manner that the pH indicator is retained on the hub in a stable condition until ready for use and in an amount that will provide a visual indication to the patient that leakage has occurred that will interfere with the proper dosage.

As shown in Fig. 6, the pH color indicator when used as the leak detector can be applied on the bottom face of the hub that can contact the skin when the pad is adhesively attached to the skin of the patient. In the illustrated embodiment, the pH indicator forms a substantially continuous leak indicator around the injection site for contacting fluid leaking from the delivery site. As shown, the pH indicator is positioned within the open area defined by the adhesive of the pad. The pH indicator is typically positioned a suitable distance from the delivery site to provide a rapid detection of leakage at the delivery site. The pH indicator can be spaced a sufficiently small distance from the injection site to avoid a false positive indication of leakage in the event a small amount of the insulin or other fluid leaks during the insertion and initial starting of the fluid delivery that will not otherwise reduce the effectiveness of the delivery dosage. In one embodiment, the pH indicator can be located a distance from the deliver site that will detect leakage in amounts of 10-100 µl. In other embodiments, the pH indicator is located a distance from the injection site that will detect leakage from the injection site in amounts of about 50 µl or more. The pH indicator can detect leakage of about 100 µl or more from the injection site. The amount of the pH indicator can be selected based on the dimensions of the delivery device, the location of the pH indicator and the leak detector, and the concentration of the pH of delivery fluid. In one embodiment, the pH indicator can be present in the leak detector in an amount of about 0.5g to about 25g. In a further embodiment, the pH indicator can be on or within the adhesive material around the delivery site.

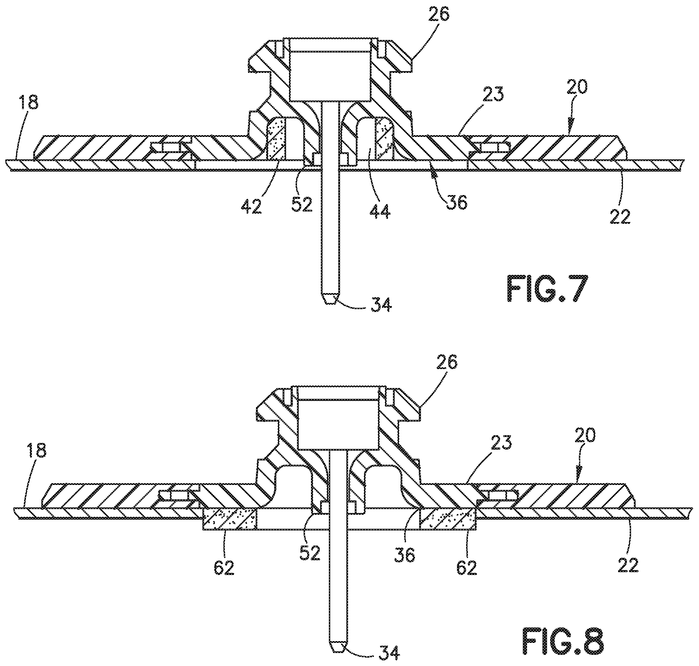

As shown in the embodiment of Fig. 7, the leak detector 42 can be an absorbent material that is capable of absorbing or wicking fluid leaking from the injection site and carrying the fluid into contact with the pH indicator. The leak detector in the embodiment of Fig. 7 and Fig. 8 refers to device, coating or structure that includes or contains the pH color indicator. The absorbent material can be impregnated, coated, or in contact with a sufficient amount of the pH indicator to provide the visual indication to the user of leakage occurring at the delivery site. In the embodiments of the invention, the pH indicator compound is included in the absorbent material of the leak detector in an amount that will be able to detect small amounts of leakage from the delivery site where the fluid from the delivery site contacts the absorbent material. The absorbent material can contain, for example, about 1-10g of the pH indicator.

The leak detector 42 has least one surface oriented to contact the fluid leaking from the infusion site and at least one surface that is visible to the patient. The infusion device, such as the hub 20, can have a window or transparent area where the leak detector can be observed by the patient. In one embodiment, the leak detector can wick or transport the fluid such as the insulin from the injection site to the surface that is visible to the patient. The pH indicator can be concentrated on the surface or area of the leak detector that is visible to the patient, such as a window or sufficiently transparent portion of the infusion device.

In the embodiment shown, the absorbent material of the leak detector 42 has a substantially annular shape surrounding the cannula 34 and cannula support post 52 to form an open area between the absorbent material 42 and the delivery site to capture the fluid that may be leaking from the delivery site. The annular shape of the leak indicator 42 defines an annular space 44 surrounding the cannula 34 within the recess 40. As shown, annular space 44 is formed between the inner wall of hub 20 and the outer surface of cannula support hub 52. The absorbent material of the leak detector 42 is spaced from the cannula a distance to define the open area between the cannula and the leak detector to capture the fluid leaking from the delivery site. The absorbent material of the leak detector 42 can have a height corresponding to the depth of the recess 40 as shown in Fig. 7. In this embodiment, the leak detector 42 contacts the skin around the delivery site so that leakage from the delivery does not escape without contacting the leak detector.

In alternative embodiments, the leak detector 42 can fill the entire recess 40 so that no gap or space is present between the cannula 34 and the leak detector 42. In another embodiment, the leak detector 42 can include an inwardly extending portion that extends between the outer wall and inner wall of annular recess 40 with the cavity 44 being formed below leak detector 42 so that the cavity 44 surrounds the cannula hub 52 and the cannula 34. The recess 40 can be provided to alleviate skin tensioning during insertion of the cannula 34. In other embodiments, the leak detector 42 occupies only a portion of the cavity 44, can be formed from segments or can be a plurality of leak detectors that are contiguous or spaced-apart.

In one embodiment, the leak indicator is formed within a portion of the hub 20 that has a cylindrical shape. The leak detector can be provided within the cylindrical portion 50 of the port 26. The leak detector is visible through the side wall of the cylindrical portion 50 of the port 26 and/or well as the top face 23 of the hub 20 to enhance visualization when a color change occurs.

In another embodiment shown in Fig. 8, the leak detector 62 can be an absorbent material coated or impregnated with the pH indicator and attached to or formed on the bottom face 36 of the hub 20 to contact the skin of the patient around the cannula 34. In this manner, fluid leaking at the delivery site is captured by the absorbent leak detector 42.

The leak indicator in the embodiments of Figs. 7 and 8 is an absorbent material containing a pH indicator as a color-changing component that is able to provide a visual color indicator in the event of leakage at the infusion site. The absorbent material can be an absorbent paper, porous fibrous material, or hydrogel containing an amount of the pH indicator that will provide a visual indication that insulin or other fluid is leaking from the delivery site in amounts that will interfere with the desired dosage or rate of delivery. The leak detector and the color change can be visible by the user through a transparent portion of the hub 20. As shown, the delivery site at the cannula penetration site is typically where leakage occurs. The leakage can be the result of an improperly inserted cannula or a cannula that has been partially or completely removed as a result of movement of the infusion set 12. In preferred embodiments of the invention, the leak indicator is positioned to provide a rapid visual indicator to the patient that leakage has occurred, thereby providing an opportunity to correct the leakage and provide the intended dosage. The leak detector 42 provides a visual color indicator of leakage through the delivery device.

In the embodiments of Figs. 7 and 8, leakage occurring at the infusion site is captured in the cavity 44 and leak detector. The fluid being delivered to the patient is absorbed by the leak detector which then reacts with one or more compounds in the leak detector to provide a visual indicator on or through the delivery device.

In one embodiment, the leak detector 42 can be made from a transparent high diffusion hydrogel having at least one compound dispersed therein that is capable of undergoing a color change when contacted with the fluid or infusion liquid. The hydrogel can be formed as a film or cylindrical shaped member having an adhesive backing on one side for attaching the hydrogel to one or more surfaces on the bottom surface of the delivery device or an infusion set. Examples of hydrogels include polyacrylamides, silicone hydrogels, crosslinked polyethylene oxide and crosslinked polyvinylpyrrolidone. The clear or transparent components of the infusion set enable visualization of the reaction by a color change through one or more parts of the infusion set.

According to the invention, the infusion liquid is an insulin formulation. The leak detector can contain at least one component that reacts with a component or compound of the insulin formulation that is not present in body fluids to avoid giving a false indication of leakage of the insulin formulation. Insulin formulations typically include insulin, hexamer zinc stabilizer preservatives, pH buffers, surfactants such as glycerol and tonicity agents such as sodium chloride. Common insulin preservatives or stabilizing agents include m-cresol, phenol, and mixtures thereof.

In other embodiments, the leak detector 42 contains a pH color indicator that can be used in combination with other compounds that react with the stabilizing agents, such as m-cresol and/or phenol, to produce a visual color change when the infusion liquid is absorbed by the leak detector. The additional color changing compounds in the leak detector are preferably non-reactive with the pH color indicator and produce a color change when reacted with the stabilizing agents of the insulin or other infusion liquid.

The additional color changing compounds can include a mixture of 4-aminoantipyrine and an oxidizing agent, such a potassium persulfate, in an effective amount to produce a color change by reacting with the stabilizing agents when the infusion liquid contacts the leak detector 42. Potassium persulfate as the oxidizing agent has been found to provide a rapid color change in the presence of phenol, m-cresol and -4-aminoantipyrine. The leak detector can also include a catalyst or enzyme to enhance the speed of the reaction and amplify the color change, such as horseradish peroxidase (HRP).

While the various embodiments illustrate the invention, it will be understood that various changes and modifications can be made without departing from the scope of the invention as defined in the appended claims.

## Claims

1. A fluid infusion set for introducing a fluid into a patient, said fluid infusion set comprising:
a cannula (34) for introducing insulin into the patient at an injection site on the patient; and
a leak detector (42) positioned surrounding the cannula (34) to contact and
detect leakage of said insulin from the injection site, said leak detector (42) producing a visible color change when in contact with the fluid leakage from the injection site to detect the leakage of said insulin from the injection site, **characterized in that** said leak detector (42) comprises a pH indicator.

2. The fluid infusion set of claim 1, wherein said hub of said delivery device has a top side and where said color change from said pH indicator (60) is visible through said top side of said hub.

3. The fluid infusion set of claim 1, wherein said pH indicator exhibits a visible color change at pH 7.0 to pH 7.8, and preferably said pH indicator (60) is bromothymol blue, phenol red, neutral red, cresol red or naphthothalein.

4. The fluid infusion set of claim 1, wherein said hub includes a fluid coupling and a bottom face with an annular recess (40) surrounding said cannula (34) and where said pH indicator is provided in said annular recess (40), and preferably said interface includes an adhesive for attaching said delivery device to the patient, where said adhesive (22) surrounds said pH indicator (60).

5. The fluid infusion set of claim 1, wherein said leak detector is an absorbent material impregnated with said pH indicator (60), and where said absorbent material is attached to said bottom side of said hub.

6. The fluid infusion set of claim 1, wherein said delivery device is configured for delivering an insulin solution, said insulin solution having a pH of about 7.0 to 7.8, and where said pH indicator (60) exhibits a visible color change at about pH 7.0 to 7.8.

7. The fluid infusion set of claim 1, wherein said leak indicator is an absorbent material impregnated with said pH indicator (60), and where said absorbent material is on said interface and visible to the patient through a top side of said hub.

8. The fluid infusion set of claim 1, wherein said hub has a fluid coupling on a top face and said bottom side has an annular recess (40) surrounding said cannula (34) to capture the fluid leaking from said injection site, and where said pH indicator (60) is provided in said recess.

9. The fluid infusion set of claim 1, wherein said insulin has a pH of about 7.0 to 7.8, and wherein said pH indicator (60) does not exhibit a color change when in contact with skin of the patient, and exhibits a color change when in contact with said insulin.

10. The fluid infusion set of claim 1, wherein said hub has an outer surface where said leak detector (42) is visible to the patient through said outer surface.

11. The fluid infusion set of claim 1, wherein said leak detector (42) is an absorbent material containing said pH indicator (60) and where said absorbent material has a first side configured for contacting fluid leakage from said injection site, and a second side visible through an outer surface of said delivery element.

## Patentansprüche

1. Fluidinfusionsset zum Einführen eines Fluids in einen Patienten, wobei das Fluidinfusionsset aufweist:
eine Kanüle (34) zum Einführen von Insulin in den Patienten an einer Injektionsstelle am Patienten; und
einen Leckdetektor (42), der um die Kanüle (34) herum positioniert ist, um mit dem aus der Injektionsstelle austretenden Insulin in Kontakt zu kommen und es zu erfassen, wobei der Leckdetektor (42) eine sichtbare Farbänderung erzeugt, wenn er mit dem aus der Injektionsstelle austretenden Fluid in Kontakt kommt, um das Austreten des Insulins aus der Injektionsstelle zu erfassen.

2. Fluidinfusionsset nach Anspruch 1, wobei der Ansatz der Abgabevorrichtung eine Oberseite hat und die Farbänderung des pH-Indikators (60) durch die Oberseite des Ansatzes sichtbar ist.

3. Fluidinfusionsset nach Anspruch 1, wobei der pH-Indikator eine sichtbare Farbänderung bei pH 7,0 bis pH 7,8 zeigt und der pH-Indikator (60) vorzugsweise Bromthymolblau, Phenolrot, Neutralrot, Kresolrot oder Naphthothalein ist.

4. Fluidinfusionsset nach Anspruch 1, wobei der Ansatz eine Fluidkopplung und eine Bodenfläche mit einer ringförmigen Aussparung (40) aufweist, welche die Kanüle (34) umgibt, und wobei der pH-Indikator in der ringförmigen Aussparung (40) vorgesehen ist, und wobei die Schnittstelle vorzugsweise einen Klebstoff zum Befestigen der Abgabevorrichtung am Patienten aufweist, wobei der Klebstoff (22) den pH-Indikator (60) umgibt.

5. Fluidinfusionsset nach Anspruch 1, wobei der Leckdetektor ein mit dem pH-Indikator (60) imprägniertes Absorptionsmaterial ist, und wobei das Absorptionsmaterial an der Unterseite des Ansatzes angebracht ist.

6. Fluidinfusionsset nach Anspruch 1, wobei die Abgabevorrichtung für die Abgabe einer Insulinlösung ausgebildet ist, wobei die Insulinlösung einen pH-Wert von etwa 7,0 bis 7,8 aufweist, und wobei der pH-Indikator (60) eine sichtbare Farbänderung bei etwa pH 7,0 bis 7,8 zeigt.

7. Fluidinfusionsset nach Anspruch 1, wobei der Leckindikator ein mit dem pH-Indikator (60) imprägniertes Absorptionsmaterial ist, und wobei sich das Absorptionsmaterial an der Schnittstelle befindet und für den Patienten durch eine Oberseite des Ansatzes sichtbar ist.

8. Fluidinfusionsset nach Anspruch 1, wobei der Ansatz eine Fluidkopplung auf einer Oberseite aufweist und die Unterseite eine ringförmige Aussparung (40) aufweist, welche die Kanüle (34) umgibt, um das aus der Injektionsstelle austretende Fluid aufzufangen, und wobei der pH-Indikator (60) in der Aussparung vorgesehen ist.

9. Fluidinfusionsset nach Anspruch 1, wobei das Insulin einen pH-Wert von etwa 7,0 bis 7,8 hat, und wobei der pH-Indikator (60) bei Kontakt mit der Haut des Patienten keine Farbänderung zeigt und bei Kontakt mit dem Insulin eine Farbänderung zeigt.

10. Fluidinfusionsset nach Anspruch 1, wobei der Ansatz eine Außenfläche aufweist, wobei der Leckdetektor (42) für den Patienten durch die Außenfläche sichtbar ist.

11. Fluidinfusionsset nach Anspruch 1, wobei der Leckdetektor (42) ein Absorptionsmaterial ist, das den pH-Indikator (60) enthält, und wobei das Absorptionsmaterial eine erste Seite hat, die so ausgebildet ist, dass sie mit Fluidleckagen aus der Injektionsstelle in Kontakt kommt, und eine zweite Seite hat, die durch eine Außenfläche des Abgabeelements sichtbar ist.

## Revendications

1. Ensemble de perfusion de fluide pour introduire un fluide chez un patient, ledit ensemble de perfusion de fluide comprenant :
une canule (34) pour introduire de l'insuline chez le patient au niveau d'un site d'injection chez le patient ; et
un détecteur de fuite (42) positionné de manière à entourer la canule (34) pour entrer en contact avec et détecter une fuite de ladite insuline du site d'injection, ledit détecteur de fuite (42) produisant un changement de couleur visible lors du contact avec la fuite de fluide du site d'injection pour détecter la fuite de ladite insuline du site d'injection, **caractérisé en ce que** ledit détecteur de fuite (42) comprend un indicateur de pH.

2. Ensemble de perfusion de fluide selon la revendication 1, dans lequel ledit raccord dudit dispositif d'administration a un côté supérieur et où ledit changement de couleur dudit indicateur de pH (60) est visible à travers ledit côté supérieur dudit raccord.

3. Ensemble de perfusion de fluide selon la revendication 1, dans lequel ledit indicateur de pH présente un changement de couleur visible à un pH égal à 7,0 jusqu'à un pH égal à 7,8, et de préférence ledit indicateur de pH (60) est le bleu de bromothymol, le rouge de phénol, le rouge neutre, le rouge de crésol ou la naphtholphthaléine.

4. Ensemble de perfusion de fluide selon la revendication 1, dans lequel ledit raccord comprend un couplage fluidique et une face inférieure avec un évidement annulaire (40) entourant ladite canule (34) et où ledit indicateur de pH est prévu dans ledit évidement annulaire (40), et de préférence ladite interface comprend un adhésif pour fixer ledit dispositif d'administration au patient, où ledit adhésif (22) entoure ledit indicateur de pH (60).

5. Ensemble de perfusion de fluide selon la revendication 1, dans lequel ledit détecteur de fuite est un matériau absorbant imprégné dudit indicateur de pH (60), et où ledit matériau absorbant est fixé audit côté inférieur dudit raccord.

6. Ensemble de perfusion de fluide selon la revendication 1, dans lequel ledit dispositif d'administration est configuré pour administrer une solution d'insuline, ladite solution d'insuline ayant un pH d'environ 7,0 à 7,8, et où ledit indicateur de pH (60) présente un changement de couleur visible à un pH d'environ 7,0 à 7,8.

7. Ensemble de perfusion de fluide selon la revendication 1, dans lequel ledit indicateur de fuite est un matériau absorbant imprégné dudit indicateur de pH (60), et où ledit matériau absorbant est sur ladite interface et visible par le patient à travers un côté supérieur dudit raccord.

8. Ensemble de perfusion de fluide selon la revendication 1, dans lequel ledit raccord a un couplage fluidique sur une face supérieure et ledit côté inférieur a un évidement annulaire (40) entourant ladite canule (34) afin de capturer le fluide s'échappant dudit site d'injection, et où ledit indicateur de pH (60) est prévu dans ledit évidement.

9. Ensemble de perfusion de fluide selon la revendication 1, dans lequel ladite insuline a un pH d'environ 7,0 à 7,8, et dans lequel ledit indicateur de pH (60) ne présente pas de changement de couleur lors du contact avec la peau du patient, et présente un changement de couleur lors du contact avec ladite insuline.

10. Ensemble de perfusion de fluide selon la revendication 1, dans lequel ledit raccord a une surface externe où ledit détecteur de fuite (42) est visible par le patient à travers ladite surface externe.

11. Ensemble de perfusion de fluide selon la revendication 1, dans lequel ledit détecteur de fuite (42) est un matériau absorbant contenant ledit indicateur de pH (60) et où ledit matériau absorbant a un premier côté configuré pour entrer en contact avec la fuite de fluide dudit site d'injection, et un second côté visible à travers une surface externe dudit élément d'administration.
